# EUROPEAN PATENT APPLICATION

(11) **EP 2 392 926 A1**
(43) Date of publication of application: **07.12.2011**
(21) Application number: 11153906.0
(22) Date of filing: 09.02.2011
(51) Int. Cl.: G01N 33/68

(54) **Urinary biomarkers in HIV infected subjects**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andreas, 68259 Mannheim (DE); Zdunek, Dietmar, 82327 Tutzing (DE)
(74) Representative: Dick, Alexander

(57) **Abstract**

The present invention relates to the field of diagnostic measures. Specifically, it is directed to a method for assessing whether an HIV infected subject who is or who will be administered a nucleotide reverse transcriptase inhibitor is susceptible to the administration of a non-nucleoside reverse transcriptase inhibitor (NNRTI), said method comprising determining the amount of L-FABP (liver fatty acid binding protein) in a sample from said subject, and comparing the amount of L-FABP to a reference amount, whereby it is assessed whether said subject is susceptible to the administration of a non-nucleoside reverse transcriptase inhibitor (NNRTI). Further encompassed are diagnostic devices and kits for carrying out the aforementioned methods.

## Description

The present invention relates to the field of diagnostic measures. Specifically, it is directed to a method for assessing whether an HIV infected subject who is or who will be administered a nucleotide reverse transcriptase inhibitor is susceptible to the administration of a non-nucleoside reverse transcriptase inhibitor (NNRTI), said method comprising determining the amount of L-FABP (liver fatty acid binding protein) in a sample from said subject, and comparing the amount of L-FABP to a reference amount, whereby it is assessed whether said subject is susceptible to the administration of a non-nucleoside reverse transcriptase inhibitor (NNRTI). Further encompassed are diagnostic devices and kits for carrying out the aforementioned methods.

Approximately 33 million people worldwide were infected with the human deficiency virus according to estimates of the WHO in 2007. This number continues to increase according to an UNAIDS report on the Global AIDS epidemic.

Kidney disorders associated with HIV have first been described in 1984 and were originally termed "aquired immune deficiency syndrome (AIDS) nephropathy and later renamed HIV-associated nephropathy (HIVAN), see Rao T.K. et al N. Engl J Med 310: 669 - 673, 1984). HIVAN has been shown to be associated with collapsing glomerulopathy and tubular microcystic disease. It has become clear that HIVAN has a strong genetic predisposition and is almost exclusively found in blacks (Cantor E.S. Arch Internal Med 151: 125 - 128, 1991, Kaufman L. et al Advances in Chronic Kidney Disease 17: 36 - 43, 2010). HIV viral proteins specifically nef and vpr are believed to play a role in HIVAN according to animal models (Zuo Y. Et al J Am Soc Nephrol 17:2832 - 2843, 2006). HIVAN has been shown to benefit from effective antiretroviral therapy.

In addition to HIVAN, a variety of kidney disorders have been linked to HIV infection, including immune complex glomerulonephritis (ICGN), IgA nephropathy, thrombotic mi-croangiopathy (TMA) and others (see, Rachakonda A.K. et al, Advances in chronic Kidney disease 17: 83 - 93, 2010).

In the past two decades highly active antiretroviral therapy (HAART) has been implemented. This therapy uses a combination of different classes of antiviral drugs to suppress viral replication. These classes of drugs comprise nucleoside reverse transcriptase inhibitors (NRTI), non-nucleoside reverse transcriptase inhibitors (NNRTI), protease inhibitors and more recently integrase and cell entry (fusion and CCR5) inhibitors. (Kalayjian R.C., Advances in chronic kidney disease 17: 59 - 71, 2010).

Nucleoside reverse transcriptase inhibitors are excreted by the kidneys with the exception of abacavir and have a favourable renal profile. The only approved nucleotide analogue is tenofovir, this drug is known to cause acute kidney injury. NNRTIs are metabolized in the liver and then excreted by the kidney and are clinically associated with minimal nephrotoxicity. Protease inhibitors are also metabolized in the liver and do also not exhibit significant renal toxicity, however may result in urolithiasis. (Jao, J. et al Advances in chronic kidney disease 17:72 - 82, 2010).

HAART has resulted in sustained suppression of HI viremia and prolonged survival and more importantly in reduction of AIDS defining events, however if such events occur potentially nephrotoxic drugs may be needed such as trimethoprim-sulfamethoxazole, antituberculosis medication, amphotericin as an antifugal drug and antiviral drugs used for systemic CMV (cytomegalovirus) or HBV(hepatitis B Virus) infections. Such nephrotoxic drugs include Ganciclovir, Cidofovir, Adefovir and Foscarnet. Also, superimposed hepatitic C virus infection was associated with kidney damage.

Another aspect of significantly improved survival of HIV infected patients are comorbidities known to be associated with kidney involvement such as diabetes mellitus, arterial hypertension and dyslipemia. The complexity of drug therapy in HIV infected individuals needs also to consider other comorbidities such as cardiovascular, metabolic, liver, neurologic, bone or other diseases (Winston J-A. Advances in chronic kidney disease 17:19-25,2010)

At this point, kidney disease was only looked at using albumin in urine and serum (plasma) markers of renal function such as creatinine or cystatin C, with the exception of Hall A.W. et al (Am J. Kidney Dis 54: 1034 - 42,2009) who studied retinol binding protein, N-acetyl-ß-D-glucosamidase (NAG), albumin and protein in urine).

Current recommendations for the treatment of HIV infection require to consider viral resistance to antiviral drugs, pill burden, dosing schedule, tolerability profile and comorbid conditions like cardiovascular or renal disease as well as with the hepatitis B and C viruses (Zolopa A.R., Antiviral Research 2010:85: 241 - 244). For reasons of convenience (one pill per day) and general tolerability one combination frequently used is efavirenz, tenofovir and emtricabine called Atripla^{®}. Tenofovir however has been shown to be associated with acute nephrotoxicity (Herlitz L.C. et al Kidney International 2010: 78: 1171 - 1177) and deterioration of renal function over time (Horberg M et al Acquir Immune Defic Syndr. 2010: 53: 62- 69). Tenofovir is excreted through tubular epithelial cells and secretion may be altered in case of ABCC2 polymorphism, in fact, acute kidney injury has been shown to be morphologically associated with acute tubular necrosis (Herlitz L.C. et al Kidney International 2010: 78: 1171 - 1177).

Urinary L-FABP has been shown to be a useful marker for tubular damage both in chronic kidney disease (Kamijo et al, Mol Cell Biochem 2006: 284) and in case of acute kidney injury after cardiac surgery (Protilla D. et al Kidney International 2008: 73: 465 - 472) and thus to provide useful information beyond kidney function markers such as creatinine and cystatin C.

Van Timmeren et al. (J. Pathol 2007; 212:209-217) reported that tubular kidney injury molecule (KIM-1) is upregulated in renal disease and is associated with renal fibrosis and inflammation. Moreover urinary KIM-1 reflects tissue Skim-1, indicating that it can be used as a non-invasive biomarker in renal disease. One advantage of KIM-1 as a urinary biomarker is the fact that its expression seems to be limited to the dysfunctional kidney (P. Devarajan, Expert Opin. Med, Diagn, (2008) 2(4):387-398).

As mentioned above, nucleotide reverse transcriptase inhibitors (in particular tenofovir) have been shown to be nephrotoxic. Therefore, there is a need in HIV patients treated with a nucleotide reverse transcriptase inhibitor of choosing a combination therapy which puts the patients at lowest risk of acute renal events.

The technical problem underlying the present invention can be seen as the provision of means and methods for reliably and efficiently assessing in a subject who is or who will be administered a nucleotide reverse transcriptase inhibitor (NTRTI) whether said subject is susceptible to further antiretroviral drugs. The technical problem is solved by the embodiments characterized in the claims and herein below.

It has been found in the context of the studies of the present invention that the combination of a nucleotide reverse transcriptase inhibitor (NTRTI) and a non-nucleoside reverse transcriptase inhibitor (NNRTI) resulted in increased renal tubular damage as compared to the combination of a nucleotide reverse transcriptase inhibitor (NTRTI) with a protease inhibitor (PI).

Accordingly, the present invention relates to a method for assessing whether an HIV infected subject who is or who will be administered a nucleotide reverse transcriptase inhibitor (NTRTI) is susceptible to the administration of a non-nucleoside reverse transcriptase inhibitor (NNRTI), said method comprising
a) determining the amount of L-FABP (liver fatty acid binding protein) in a sample from said subject, and
b) comparing the amount of L-FABP to a reference amount, whereby it is assessed whether said subject is susceptible to the administration of a non-nucleoside reverse transcriptase inhibitor (NNRTI).

Preferably, it is assessed whether said subject is susceptible to the administration of a non-nucleoside reverse transcriptase inhibitor by carrying out the further step of c) assessing whether said subject is susceptible to the administration of a non-nucleoside reverse transcriptase inhibitor based on the result of the comparison carried out in step b).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "subject" as used herein, preferably, relates to a human. The subject according to the present invention is, preferably, infected with the human immunodeficiency virus (HIV). The human immunodeficiency virus is a retrovirus that may cause acquired immunodeficiency syndrome (AIDS), a condition which may lead to life-threatening opportunistic infections. Preferably, the subject is infected with HIV-2. More preferably, the subject is infected with HIV-1.

Preferably, the subject is an individual without any apparent renal disorder. Renal disorders are known to the person skilled in the art. In this context, the term "renal disorder" is considered to relate to any disease, injury, or dysfunction of the kidney or affecting the kidney, more particularly affecting the capacity of the kidney for waste removal and/or ultrafiltration. Examples for renal disorders include congenital disorders and acquired disorders. Examples for congenital renal disorders include congenital hydronephrosis, congenital obstruction of urinary tract, duplicated ureter, horseshoe kidney, polycystic kidney disease, renal dysplasia, unilateral small kidney. Examples for acquired renal disorders include diabetic or analgesic nephropathy, glomerulonephritis, hydronephrosis (the enlargement of one or both of the kidneys caused by obstruction of the flow of urine), interstitial nephritis, kidney stones, kidney tumors (e.g., Wilms tumor and renal cell carcinoma), lupus nephritis, minimal change disease, nephrotic syndrome (the glomerulus has been damaged so that a large amount of protein in the blood enters the urine. Other frequent features of the nephrotic syndrome include swelling, low serum albumin, and high cholesterol), pyelonephritis, renal failure (e.g., acute renal failure and chronic renal failure). Renal disorders can be diagnosed by any means known and deemed appropriate. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. The GFR was originally estimated (the GFR can never be determined, all calculations derived from formulas such as the Cockgroft Gault formula of the MDRD formula deliver only estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 ml/min. Thus, it is particularly contemplated that the GFR of the subject as referred to herein is within this range. Moreover, the subject as referred to in the context of the method present invention, preferably, has a blood creatinine level (in particular a serum creatinine level) of lower than 0.9 mg/dl, more preferably of lower than 1.1 1 mg/dl and most preferably of lower than 1.3 mg/dl.

It is, preferably, envisaged that the subject shall suffer from diabetes mellitus (in particular type 2 diabetes). Moreover, it is envisaged that the subject may have a co-infection with HCV (hepatitis C virus) or HBV (hepatitis B virus). Moreover, the subject may suffer from heart failure or not.

Antiretroviral medicaments may also be used for preexposure prophylaxis (see Morbidity and Mortality Weekly Reports 2011;60(03);65-68). Therefore, is it also contemplated that the subject is not infected with HIV. In this case, the subject, preferably, is a subject having an increased risk of infection with the HI-virus.

In the context of the present invention, the subject is or will be administered a nucleotide reverse transcriptase inhibitor, and, thus, is treated with a nucleotide reverse transcriptase inhibitor when obtaining the sample or will be treated with nucleotide reverse transcriptase inhibitor after the sample has been obtained.

Accordingly, the method of the present invention allows for treatment decisions in a subject i) who is not treated with a nucleotide reverse transcriptase inhibitor at the time at which the sample is obtained and who, however, shall be treated with a nucleotide reverse transcriptase inhibitor after obtaining the sample from said subject, or in ii) a subject who is treated with a nucleotide reverse transcriptase inhibitor at the time at which the sample is obtained.

In the first case (i), decisions shall be made for a subject in whom the administration of nucleotide reverse transcriptase inhibitor shall be initiated. Preferably, the therapy is initiated after the sample to be analyzed in the context of the present invention has been obtained. More preferably, the therapy is initiated not more than 30 days, not more than 20 days, even more preferably not more than 15 days, and most preferably, not more than 7 days after said sample has been obtained.

In the latter case (ii), decisions shall be made for a subject in whom the treatment with a nucleotide reverse transcriptase inhibitor shall be continued. Preferably, said subject has been treated with a nucleotide reverse transcriptase inhibitor at least one month, at least three months, at least six months, at least twelve months, at least one year, and more preferably, at least two years before the sample to be analyzed in the context of the present invention has been obtained.

As set forth above, the subject is or will be treated with a nucleotide reverse transcriptase inhibitor. As used herein, the term "nucleotide reverse transcriptase inhibitor" (abbreviated "NTRTI", frequently also referred to as "Nucleotide analog reverse transcriptase inhibitors), preferably, refers to a member of a class of drugs that are chemical analogs of a nucleoside 5 '-monophosphate that inhibit a reverse transcriptase or polymerase enzyme and therefore the ability of a virus to infect a host cell. A nucleotide reverse transcriptase inhibitor may be a chemical analog of a ribonucleotide or a deoxyribonucleotide.

Preferably, the nucleotide reverse transcriptase inhibitor is adefovir (in particular adefovir dipivoxil). More preferably, the nucleotide reverse transcriptase inhibitor is tenofovir. Usually, Tenofovir is administered as the prodrug form tenofovir disoproxil fumarate (trade name, e.g., Viread^{®}).

The daily dosage of the nucleotide reverse transcriptase inhibitor and, in particular, of tenofovir or tenofovir disoproxil fumarate is, preferably, 50 to 750 mg, more preferably, 100 to 400 mg, event more preferably, 200 to 400 mg, and most preferably 300 mg. Preferably, the nucleotide reverse transcriptase inhibitor is administered orally.

The method of the present invention allows of identifying a subject being susceptible to the administration of a non-nucleoside reverse transcriptase inhibitor:

Non-nucleoside reverse transcriptase inhibitors (NNRTIs) are antiretroviral drugs used in the treatment of human immunodeficiency virus. Like nucleoside reverse transcriptase and nucleotide reverse transcriptase inhibitors, NNRTIs inhibit reverse transcriptase which controls the replication of the genetic material of HIV. In contrast to nucleoside reverse transcriptase and nucleotide reverse transcriptase inhibitors, NNRTIs have a cdifferent mode of action. NNRTIs block reverse transcriptase by binding at a different site on the reverse transcriptase. NNRTIs are not incorporated into the viral DNA, rather they inhibit the movement of protein domains of reverse transcriptase that are needed to carry out the process of DNA synthesis. NNRTIs are therefore classified as non-competitive inhibitors of reverse transcriptase.

Preferred non-nucleoside reverse transcriptase inhibitors (NNRTI) include efavirenz, nevirapine, delavirdine, etravirine. Of these, efavirenz und nevirapine are the most preferred NNRTIs.

Efavirenz is, preferably, administered with a daily dosage of 100 to 1000 mg, more preferably, with a daily dosage of 400 to 800 mg, and most preferably, with daily dosage of 600 mg.

Nevirapine is, preferably, administered with a daily dosage of 200 to 600 mg, more preferably, with a daily dosage of 400 to 800 mg, and most preferably, with daily dosage of 400 mg.

By "assessing whether an HIV infected subject is susceptible to the administration of a non-nucleoside reverse transcriptase inhibitor (NNRTI)", a subject shall be identified who is susceptible to the administration of a non-nucleoside reverse transcriptase inhibitor (NNRTI) or not.

Preferably, a subject who is susceptible to the administration of a non-nucleoside reverse transcriptase inhibitor (NNRTI) is a subject who is not at risk, or at low risk of suffering from adverse side effects of the therapy. Preferably, a subject who is not susceptible to the administration of a non-nucleoside reverse transcriptase inhibitor (NNRTI) is at risk or at increased risk of suffering from adverse side effects of the therapy. The adverse side effects are, preferably, damage of the renal tubules, development of chronic kidney disease, or, more preferably, acute kidney injury.

It is to be understood that the assessment whether a subject as referred to herein is susceptible to the administration of a non-nucleoside reverse transcriptase inhibitor (NNRTI) is usually not intended to be correct for 100% of the subjects to be assessed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, 0.001 or 0.0001.

Acute kidney injury (AKI) is a rapid loss of kidney function. Depending on its severity, AKI may lead to a number of complications, including metabolic acidosis, high potassium levels, uremia, changes in body fluid balance, and effects to other organ systems. Management includes supportive care, such as renal replacement therapy, as well as treatment of the underlying disorder. As used herein, the term "acute kidney injury", preferably, refers an absolute increase (preferably, within 48 hours) in the serum creatinine concentration of ≥0.3 mg/dL (26.4 micromol/L) from baseline, or ≥ 50 percent increase in the serum creatinine.

In the context of the present invention, the term "damage of renal tubules", preferably, refer to epithelial injury in renal tubule cells. In tubular damage, tubule cells may become dysfunctional, in general by necrosis, and die. However, tubular epithelium regeneration is possible after ischemia and even after necrosis, referred to as "tubular repair" in the context of the present invention.

The term "sample" refers to a sample of a body fluid, to a sample of separated cells or to a sample from a tissue or an organ. Samples of body fluids can be obtained by well known techniques and include, preferably, samples of blood, plasma, serum, or, more preferably, urine. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The term "liver-type fatty acid binding protein" (L-FABP, frequently also referred to as FABP-1, herein also referred to as U-L-FABP or "urinary liver-type fatty acid binding protein"), preferably, relates to a polypeptide that is encoded by the FABP1 gene. The term, preferably, relates to the human L-FABP polypeptide. L-FABP belongs to a family of small, highly conserved, cytoplasmic proteins that bind long-chain fatty acids and other hydrophobic ligands. Liver-type fatty acid binding protein is an intracellular carrier protein of free fatty acids that is, e.g., expressed in the proximal tubules of the human kidney. Kamijo et al. (Urinary liver-type fatty acid binding protein as a useful biomarker in chronic kidney disease. MoI. Cell Biochem. 2006; 284) reported that urinary excretion of L-FABP may reflect various kind of stresses that cause tubular interstitial damage and may be a useful clinical marker of the progression of chronic renal disease. The sequence of human L-FABP is e.g. disclosed by Chan et al. (Human liver fatty acid binding protein cDNA and amino acid sequence. Functional and evolutionary implications J. Biol. Chem. 260 (5), 2629-2632 (1985)), or GenBank Acc. Number M10617.1 (DNA sequence) and AAA52419.1 (protein sequence). Genbank is available from the NCBI, USA. Further, the term "L-FABP" encompasses variants of said specific human L-FABP. Such variants have at least the same essential biological and immunological properties as the specific L-FABP polypeptide. Variants are deemed to share the same essential biological and immunological properties if they are detectable by the same specific assays referred to in this specification, e.g., by ELISA assays using polyclonal or monoclonal antibodies specifically recognizing the said L-FABP polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific L-FABP polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific L-FABP polypeptides or the aforementioned types of variants as long as these fragments have the essential immunological and biological properties as referred to above. Such fragments may be, e.g., degradation products or splice variants of the L-FABP polypeptides. Further included are variants which differ due to posttranslational modifications such as phosphorylation or myristylation.

Determining the amount of L-FABP or any other peptide or polypeptide referred to in this specification (e.g. KIM-1, see below) relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and noncovalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein, preferably, encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a cardiac disease is an acute inflammation-associated cardiac disease or an acute inflammation-independent cardiac disease. Therefore, the reference amount is to be chosen so that either a difference or a similarity in the compared amounts allows for assessing whether a subject is susceptible to the administration of a non-nucleoside reverse transcriptase inhibitor (NNRTI) and, thus, for identifying those test subjects which belong into the group of subjects which are susceptible to the administration of a non-nucleoside reverse transcriptase inhibitor (NNRTI) or not.

Accordingly, the term "reference amount" as used herein, preferably, refers to an amount which allows assessing whether subject who is or who will be administered a nucleotide reverse transcriptase inhibitor is susceptible to the administration of a non-nucleoside reverse transcriptase inhibitor (NNRTI). Accordingly, the reference may be derived, in principle, from:
(i) a subject or a group of subjects known to be susceptible to the administration of a non-nucleoside reverse transcriptase inhibitor (NNRTI), and/or
(ii) a subject or a group of subjects known not to be susceptible to the administration of a non-nucleoside reverse transcriptase inhibitor (NNRTI).

Preferably, said subject (subjects) in (i) or (ii) is (are) administered or will be administered a nucleotide reverse transcriptase inhibitor as described elsewhere herein. Also preferably, the subject/subjects is (are) infected with HIV

Preferably the following is applied as a diagnostic algorithm:

Preferably, an essentially identical or a decreased amount of L-FABP (as compared to the reference amount) indicates that the subject is susceptible the administration of a non-nucleoside reverse transcriptase inhibitor (NNRTI), if a reference amount is used which is derived from a subject or group of subjects known to be susceptible to the administration of a non-nucleoside reverse transcriptase inhibitor (NNRTI).

Preferably, an essentially identical or an increased amount of L-FABP (as compared to the reference amount) is indicative for a subject not being susceptible to the administration of said non-nucleoside reverse transcriptase inhibitor (NNRTIs), if the reference amount is derived from an HIV infected subjects of a group of HIV infected subjects known not to be susceptible to the administration of a non-nucleoside reverse transcriptase inhibitor (NNRTIs).

The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts can be calculated for a cohort of subjects as specified above based on the average or mean values for a given biomarker by applying standard statistically methods. In particular, accuracy of a test such as a method aiming to assess a condition, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being susceptible to the administration of a non-nucleoside reverse transcriptase inhibitor (NNRTI) or those which are not susceptible to the administration of a non-nucleoside reverse transcriptase inhibitor (NNRTI), preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject not being susceptible to the administration of a non-nucleoside reverse transcriptase inhibitor (NNRTI) (i.e. a rule out) whereas an optimal specificity is envisaged for a subject to be assessed as being susceptible to the administration of a non-nucleoside reverse transcriptase inhibitor (NNRTI) (i.e. a rule in).

As set forth above, the sample is, preferably, urine. Preferably, the amount of the L-FABP in said urine sample is normalized to the amount of creatinine in said urine sample The normalization, preferably, allows to account for creatinine clearance and urine flow.

Preferably, the median value determined in Table 4 may be also used as a basis for establishing thresholds. Preferably, said threshold for L-FABP for a subject not being susceptible to the administration of a non-nucleoside reverse transcriptase inhibitor is within the range of the 50th percentile to the 75th percentile of the subjects being treated with both a nucleotide reverse transcriptase inhibitor (NTRTI) and a non-nucleoside reverse transcriptase inhibitor (NNRTI). Accordingly, a preferred threshold is within the range of about 9.765 µg L-FABP/g urinary Creatinine to about 16.118 µg L-FABP/g Creatinine. A particularly preferred threshold is 16.118 µg L-FABP/g Creatinine.

Preferably, an essentially identical or an increased amount of L-FABP (normalized to urinary creatinine) as compared to the aforementioned threshold is indicative for a subject not being susceptible to the administration of said non-nucleoside reverse transcriptase inhibitor (NNRTIs).

In principle, the present invention relates to a method for assessing whether an HIV infected subject who is or who will be administered a nucleotide reverse transcriptase inhibitor (NTRTI) is susceptible to the administration of a non-nucleoside reverse transcriptase inhibitor (NNRTI) based on a comparison of the amount of the biomarker L-FABP in a sample of said subject to a reference.

Advantageously, it has been found in the studies underlying the present invention that amount of L-FABP in a body fluid such as urine, blood, plasma or serum can serve as a biomarker allowing for assessing whether a subject who is or who will be administered a nucleotide reverse transcriptase inhibitor (NTRTI) is susceptible to the administration of a non-nucleoside reverse transcriptase inhibitor (NNRTI). It was found that L-FABP below a certain threshold level indicates that the subject is susceptible to the treatment with a non-nucleoside reverse transcriptase inhibitor (NNRTI), whereas L-FABP above a certain threshold level indicates that the subject is not susceptible to the treatment with a non-nucleoside reverse transcriptase inhibitor (NNRTI). This subject, preferably, is at increased risk of suffering from adverse side effects as specified elsewhere herein, e.g. of suffering from acute kidney injury, if the therapy with said NNRTI is initiated or continued.

The identification of subjects being susceptible to a therapy including the administration of a non-nucleoside reverse transcriptase inhibitor (NNRTI), is important since it has been shown that subjects that are treated with both a non-nucleoside reverse transcriptase inhibitor (NNRTI) and nucleotide reverse transcriptase inhibitor (NTRTI) have significantly increased L-FABP levels as compared to the other combination therapies including the administration of a nucleotide reverse transcriptase inhibitor (NTRTI), see Examples. The increased levels of L-FABP indicate tubular renal damage and, thus, a risk of adverse side effects such as acute kidney injury. Therefore, only subjects with a level of L-FABP below a certain threshold or (essentially identical to said threshold) shall be additionally (i.e. in addition to a NTRTI, in particular, to tenofovir) administered a non-nucleoside reverse transcriptase inhibitor (NNRTI).

Interestingly, it was found that subjects treated with a nucleotide reverse transcriptase inhibitor (NTRTI) and a protease inhibitor (PI) had significantly lower levels of L-FABP as compared to subjects treated with a nucleotide reverse transcriptase inhibitor (NTRTI) and a non-nucleoside reverse transcriptase inhibitor (NNRTI). This indicates no tubular damage or a lower extend of tubular damage.

Thus, a subject who is not susceptible to the administration of said non-nucleoside reverse transcriptase inhibitor (NNRTIs) is, preferably, susceptible to the administration of a protease inhibitor.

The term "protease inhibitor" as used herein, preferably, refers to an inhibitor of the HIV protease. Said protease is an enzyme involved in the proteolytic cleavage of viral polyprotein precursors into the individual functional proteins.

Preferred protease inhibitors include saquinavir (see, e.g. US5,196,438), ritonavir, indinavir, nelfinavir, amprenavir, lopinavir, atazanavir, fosamprenavir, tipranavir, and darunavir. Particularly preferred protease inhibitors are listed in the Examples section.

In a preferred embodiment of method of the present invention, the method further comprises the determination of the amount of KIM-1 (kidney injury molecule 1) in a sample from said subject, the determination/calculation of a ratio of the amount of L-FABP and the amount of KIM-1, and comparing the, thus, determined/calculated ratio to a reference ratio.

Based on the comparison of a ratio of the amount of L-FABP and the amount of KIM-1 to a reference ratio, it is assessed whether an HIV infected subject who is or who will be administered a nucleotide reverse transcriptase inhibitor (NTRTI) is susceptible to the administration of a non-nucleoside reverse transcriptase inhibitor (NNRTI).

Preferably, if the a ratio of the amount of L-FABP and the amount of KIM-1 to a reference ratio is determined, preferably, the step of comparing the amount of L-FABP to a reference amount as described above can be omitted (and, thus, is optional).

Preferably, the amounts of KIM-1 and L-FABP are determined in the same sample. However, it is also contemplated that the amounts are determined in different samples provided that the samples are obtained in a close temporal proximity (particularly, within 3 hours, 6 hours, or 24 hours).

The sample, preferably, is urine if also the amount of KIM-1 is determined.

The term "determining a ratio" as used herein means calculating a ratio of the amount of L-FABP to the amount of KIM-1, or vice versa.

The reference ratio is, preferably, derived from the subject or group of subjects as described above in the context of the definition of the term "reference amount". How to calculate such a ratio is well known in the art.

If the ratio is the ratio of the amount of L-FABP to the amount of KIM-1, the following applies:

Preferably, an essentially identical or a decreased ratio as compared to the reference ratio indicates that the subject is susceptible the administration of a non-nucleoside reverse transcriptase inhibitor (NNRTI), if a reference ratio is used which is derived from a subject or group of subjects known to be susceptible to the administration of a non-nucleoside reverse transcriptase inhibitor (NNRTI).

Preferably, an essentially identical or an increased ratio (as compared to the reference ratio) is indicative for a subject not being susceptible to the administration of said non-nucleoside reverse transcriptase inhibitor (NNRTIs), if the reference ratio is derived from an HIV infected subjects of a group of HIV infected subjects known not to be susceptible to the administration of a non-nucleoside reverse transcriptase inhibitor (NNRTIs).

The further determination of the amount of KIM-1 in a sample from the test subject, and the determination of a reference ratio is advantageous, since it increases the accuracy of the method of the present invention (see Examples).

Preferably, the median value for the ratio of L-FABP to KIM-1 determined in Table 4 may be also used as a basis for establishing thresholds. Preferably, said threshold for L-FABP for a subject not being susceptible to the administration of a non-nucleoside reverse transcriptase inhibitor is within the range of the 50^{th} percentile to the 75^{th} percentile of the subjects being treated with both a nucleotide reverse transcriptase inhibitor (NTRTI) and a non-nucleoside reverse transcriptase inhibitor (NNRTI).

Accordingly, a preferred threshold is within the range of about 10,82 to about 43,96. A particularly preferred threshold is 43,96.

Preferably, an essentially identical or an increased ratio of L-FABP to KIM-1 (with respect so the aforementioned thresholds) is indicative for a subject not being susceptible to the administration of a non-nucleoside reverse transcriptase inhibitor (NNRTIs).

KIM-1 (kidney injury molecule-1, frequently also referred to as "Hepatitis A virus cellular receptor 1" or "HAVcr-1") is a type I transmembrane glycoprotein expressed on dedifferentiated renal proximal tubule epithelial cells undergoing regeneration after toxic or ischemic injury. The extracellular domain of KIM-1 is composed of an immunoglobulin-like domain topping a long mucin-like domain, a structure that points to a possible role in cell adhesion by homology to several known adhesion proteins. Two splice variants (a and b), of the human KIM-1 having identical extracellular domains, differ in their cytoplasmic domains and tissue distributions. A proteolytically processed domain of KIM-1 is easily detected in the urine soon after acute kidney injury (AKI) so that KIM-1 performs as an acute kidney injury urinary biomarker (Expert Opin. Med. Diagn. (2008) 2 (4): 387-398). KIM-1 as used herein, preferably, refers to human KIM-1. More preferably, it refers to the human KIM-1 polypeptide. The amino acid sequence of the human KIM-1 polypeptide can be assessed via Genbank, see Q96D42.2 GI:73919877.

Moreover, the term "KIM-1" encompasses variants of said specific human KIM-1. Such variants have at least the same essential biological and immunological properties as the specific KIM-1 polypeptide. Variants are deemed to share the same essential biological and immunological properties if they are detectable by the same specific assays referred to in this specification, e.g., by ELISA assays using polyclonal or monoclonal antibodies specifically recognizing the said KIM-1 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific KIM-1 polypeptides. The degree of identity between two amino acid sequences can be determined as described elsewhere herein. Variants referred to above may be allelic variants or any other species specific homologs, paralogs, or orthologs. Moreover, the variants referred to herein include fragments of the specific KIM-1 polypeptides or the aforementioned types of variants as long as these fragments have the essential immunological and biological properties as referred to above. Such fragments may be, e.g., degradation products or splice variants of the KIM-1 polypeptide. Further included are variants which differ due to posttranslational modifications such as phosphorylation or myristylation.

It is to be understood that the definitions and explanations of the terms made above and below apply accordingly for all embodiments described in this specification and the accompanying claims.

Moreover, the present invention relates to a method for monitoring a therapy comprising the administration of nucleotide reverse transcriptase inhibitor in an HIV infected subject, said method comprising the steps of
a) determining the amount of L-FABP (liver fatty acid binding protein) in a first sample from said subject,
b) determining the amount of L-FABP (liver fatty acid binding protein) in a second sample from said subject, said second sample being obtained after said first sample, and
c) comparing the amount of L-FABP in said first sample to the amount in said second sample, whereby said therapy comprising the administration of nucleotide reverse transcriptase inhibitor in said subject is monitored.

The term "monitoring" as referred to above, preferably, relates to keeping track of changes of the condition of renal tubules. Renal tubules are small structures in the kidney that filter the blood and produce the urine. Preferably, said changes are associated with (i.e. in a causal and/or temporal relationship with) the therapy comprising the administration of nucleotide reverse transcriptase inhibitor (and, if also a NNRTI is administered, said changes are, preferably, associated with the therapy comprising the administration of said NNRTI).

Monitoring includes comparing the condition of the renal tubules as reflected by the amount of the biomarker in a first sample taken at a first time point to the condition of the renal tubules reflected by the amount of the biomarker in a second sample taken at a second time point. The condition may become worse and, thus, there may be progression of damage of the renal tubules, if the amount of the biomarker increases whereas there is amelioration and, thus, improvement of the condition if the biomarker decreases. If no change is observed, i.e. an essentially identical amount is determined in the first and the second sample, the condition is unchanged. An essentially identical amount is determined if no statistically significant change in the amount is determined between the first and the second sample. Whether the amounts are essentially identical can be determined by the skilled artisan without further ado. Preferably, a change, i.e. increase or decrease is statistically significant if the amounts differ by at least about 5%, at least about 10%, at least about 15%, at least about 20%, at least about 25% or at least about 50%. Accordingly, an increase of at least about 5%, at least about 10%, at least about 15%, at least about 20%, at least about 25% or at least about 50% in the second sample from the subject as compared to the first sample indicates that the condition of the renal tubules has worsened. Preferably, tubular damage has increased. Again, it is to be understood that the aforementioned method, preferably, allows monitoring in a statistically significant portion of subjects to be investigated but not necessarily in all analyzed subjects.

The therapy comprising the administration of nucleotide reverse transcriptase inhibitor has been described elsewhere herein. In a preferred embodiment of the aforementioned method, the therapy further comprises the administration of a non-nucleoside reverse transcriptase inhibitor (NNRTI). Preferred non-nucleoside reverse transcriptase inhibitors are described elsewhere herein (see, e.g., Examples section).

The first sample shall be obtained before the second sample. Preferably, it is obtained before, and, more preferably, after the therapy comprising the administration of nucleotide reverse transcriptase inhibitor has been initiated. If the sample is obtained before initiating the said therapy, the sample is preferably obtained not more than one month, more preferably, not more than two weeks, even more preferably, not more than one week, and most preferably, not more than one day before initiating the said therapy.

The "second sample" is, preferably, understood as a sample which is obtained in order to reflect a change of the amount of the biomarker as compared to the amount of the respective marker in the first sample. The second sample shall be obtained after the first sample. Preferably, the second sample is not obtained too early after the first sample (in order to observe a sufficiently significant change to allow for monitoring). Preferably, the second sample is obtained 2 weeks, 1 month, 2 months, 4 months, 6 months, 8 months, or 12 months after said first sample. It is particularly preferred to obtain said second sample 1 month or 2 months after said first sample.

In a preferred embodiment the aforementioned method, the method comprises the additional determination of KIM-1 (kidney injury molecule 1). Accordingly, the present invention also relates to a method for monitoring a therapy comprising the administration of nucleotide reverse transcriptase inhibitor in an HIV infected subject, said method comprising the steps of
a) determining the amount of L-FABP and KIM-1 in a first sample from said subj ect,
b) determining the amount of L-FABP and KIM-1 in a second sample from said subject, said second sample being obtained after said first sample, and
c) determining a first ratio of the amount of L-FABP and KIM-1 in said first sample,
d) determining a second ratio of the amount of L-FABP and KIM-1 in said second sample, and
e) comparing the first ratio to the second ratio, whereby said therapy comprising the administration of nucleotide reverse transcriptase inhibitor in said subject is monitored.

Preferably, the sample is urine.

Preferably, the amounts of KIM-1 and L-FABP are determined in the same sample in steps a) and b). However, it is also contemplated that the amounts are determined in different samples provided that the samples are obtained in a close temporal proximity (see above).

Of course, also the ratio of the amount KIM-1 to the amount of L-FABP can be determined.

If the ratio is the ratio of the amount of L-FABP to the amount of KIM-1, the following applies:

An increase of the ratio in the second sample as compared to the first samples indicates that the condition of the renal tubules became worse, and thus, increased damage of the renal tubules. If no change is observed, i.e. an essentially identical ratio is determined in the first and the second sample, the condition is unchanged. An essentially identical ratio is determined if no statistically significant change in the amount is determined between the first and the second sample. Whether the ratios are essentially identical can be determined by the skilled artisan without further ado. Preferably, a change is statistically significant if the ratios differ by at least about 5%, at least about 10%, at least about 15%, at least about 20%, at least about 25% or at least about 50%. Accordingly, an increase of the ratio of at least about 5%, at least about 10%, at least about 15%, at least about 20%, at least about 25% or at least about 50% in the second sample from the subject as compared to the first sample indicates that the condition of the renal tubules has worsened.

Moreover, the present invention relates to a method for assessing whether an HIV patient is susceptible to the administration of nephrotoxic medication, said method comprising the steps of
a) determining the amount of U-L-FABP (urinary liver fatty acid binding protein) in a urine sample from said patient,
b) comparing the amount of U-L-FABP to a reference amount, and
c) assessing whether said HIV patient is susceptible to nephrotoxic medication.

Preferably, it is assessed whether said subject is susceptible to nephrotoxic medication by carrying out the further step of c) assessing whether said subject is to nephrotoxic medication based on the result of the comparison carried out in step b).

A subject who is susceptible to nephrotoxic medication, preferably, is a subject who is not at risk of adverse side effects caused by said medication. The adverse side effect is preferably, damage of the renal tubules, development of chronic kidney disease, or, more preferably, acute kidney injury.

Preferably, the nephrotoxic medication is selected from the group consisting of trimethoprim/sulfamethoxazole, antituberculosis medication (in particular rifampicin), amphotericin B (preferably, as an antifugal drug), and antiviral drugs used for systemic CMV (cytomegalovirus) or HBV(hepatitis B Virus) infections.

Trimethoprim/sulfamethoxazole (also referred to as Co-trimoxazole, (abbreviated SXT, TMP-SMX, TMP-SMZ or TMP-sulfa) is a sulfonamide antibiotic combination of trimethoprim and sulfamethoxazole. It is frequently used for the treatment of a variety of bacterial infections. Moreover, it may be used in immunocompromised HIV patients as Pneumocystis jiroveci pneumonia prophylaxis.

Amphotericin B is a polyene antifungal drug, for systemic fungal infections. Preferably, the drug is administered intravenously.

Preferred antiviral drugs used for systemic CMV (cytomegalovirus) or HBV (hepatitis B Virus) infections are selected from the group consisting of ganciclovir, cidofovir, adefovir and foscarnet.

In a preferred embodiment the aforementioned method according to the present invention the method further comprises the steps of determining the amount of KIM-1 (kidney injury molecule 1) in a sample from said subject, determining a ratio of the amount of L-FABP and the amount of KIM-1, and comparing the, thus, determined ratio to a reference ratio. Based on the comparison of a ratio of the amount of L-FABP and the amount of KIM-1 to a reference ratio, it is assessed whether an HIV infected subject is susceptible to the administration of nephrotoxic medication.

Preferably, if the a ratio of the amount of L-FABP and the amount of KIM-1 to a reference ratio is determined, preferably, the step of comparing the amount of L-FABP to a reference amount as described above can be omitted (and, thus, is optional).

Preferably, the amounts of KIM-1 and L-FABP are determined in the same sample. However, it is also contemplated that the amounts are determined in different samples provided that the samples are obtained in a close temporal proximity (see elsewhere herein).

If the further steps as described above are carried out, the sample, preferably, is urine.

Preferred reference amounts and reference ratios are specified elsewhere herein.

Preferably, the reference amount is derived from a subject or group of subjects known to be susceptible to the administration of nephrotoxic medication. Preferably, an essentially identical or a decreased amount of L-FABP (as compared to the reference amount) indicates that the subject is susceptible the administration of nephrotoxic medication, if said reference amount is used.

Also preferably, the reference amount is derived from a subject or group of subjects known not to be susceptible to the administration of nephrotoxic medication. Preferably, an essentially identical or an increased amount of L-FABP (as compared to the reference amount) is indicative for a subject not being susceptible to the administration of nephrotoxic medication, if said reference amount is used.

The reference ratio is, preferably, derived from the subject or group of subjects as described above in the context of the definition of the term "reference amount". How to calculate such a ratio is well known in the art.

If the ratio is the ratio of the amount of L-FABP to the amount of KIM-1, the following applies:

Preferably, an essentially identical or a decreased ratio as compared to the reference ratio indicates that the subject is susceptible the administration of nephrotoxic medication, if a reference ratio is used which is derived from a subject or group of subjects known to be susceptible to the administration of nephrotoxic medication.

Preferably, an essentially identical or an increased ratio (as compared to the reference ratio) is indicative for a subject not being susceptible to the administration nephrotoxic medication, if the reference ratio is derived from an HIV infected subjects of a group of HIV infected subjects known not to be susceptible to the administration of nephrotoxic medication.

In a further preferred embodiment of the method of the present invention, TGFß-1, BMP2 and/or BMP7 is (are) determined in addition or, preferably, instead of L-FABP.

The present invention also relates to the use of the biomarker L-FABP or of a detection agent that specifically binds thereto in a sample of a subject who is or who will be administered a nucleotide reverse transcriptase inhibitor (NTRTI) for assessing whether said subject is susceptible to the administration of a non-nucleoside reverse transcriptase inhibitor (NNRTI).

In a preferred embodiment, the biomarkers L-FABP and KIM-1, or detection agents that specifically bind thereto are used.

The present invention also relates to the use of the biomarker L-FABP or of a detection agent that specifically binds thereto in a sample of a subject for monitoring a therapy comprising the administration of nucleotide reverse transcriptase inhibitor.

In a preferred embodiment, the biomarkers L-FABP and KIM-1, or detection agents that specifically bind thereto are used.

The present invention also relates to the use of the biomarker L-FABP or of a detection agent that specifically binds thereto in a sample of a subject who is or who will be administered a nucleotide reverse transcriptase inhibitor (NTRTI) for assessing whether said subject is susceptible to the administration of nephrotoxic medication.

In a preferred embodiment of the use of the present invention, the biomarkers L-FABP and KIM-1, or detection agents that specifically bind thereto are used.

The term "detection agent" as used herein refers to an agent which is capable of specifically recognizing and binding to a biomarker as referred to herein (L-FABP or KIM-1) present in a sample. Moreover, the said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.

The present invention also encompasses a device adapted for carrying out the method of the present invention, comprising
a)an analyzing unit comprising a detection agent which specifically binds to L-FABP, said unit being adapted for determining the amount of L-FABP in a sample of a subject who is or who will be administered a nucleotide reverse transcriptase inhibitor (NTRTI); and
b)an evaluation unit for comparing the determined amount with a reference amount whereby it can be assessed whether said subject is susceptible to the administration of a non-nucleoside reverse transcriptase inhibitor (NNRTI), said unit comprising a database with reference amount values derived from a subject or group of subjects as defined elsewhere herein and a computer-implemented algorithm for carrying out a comparison as specified elsewhere herein (see the section in which the corresponding method is described).

In a preferred embodiment, the device also comprises a detection agent which specifically binds to a KIM-1 adapted for determining the amount of KIM-1. Preferably, the evaluation unit (b) comprised by said device is for calculating a ratio of L-FABP to KIM-1 and for comparing the determined ratio with a reference ratio whereby it can be assessed whether said subject is susceptible to the administration of a non-nucleoside reverse transcriptase inhibitor (NNRTI), said unit comprising a database with reference ratios values derived from a subject or group of subjects as defined elsewhere herein and a computer-implemented algorithm for carrying out a comparison as specified elsewhere herein (see the section in which the corresponding method is described).

The present invention also encompasses a device adapted for assessing whether an HIV patient who or will be administered a nucleotide reverse transcriptase inhibitor (NTRTI) is susceptible to nephrotoxic medication, comprising
a)an analyzing unit comprising a detection agent which specifically binds to L-FABP, said unit being adapted for determining the amount of L-FABP in a sample of said subject, and
b)an evaluation unit for comparing the determined amount with a reference amount whereby it can be assessed whether said subject is susceptible to the administration of nephrotoxic medication, said unit comprising a database with reference amount values derived from a subject or group of subjects as defined elsewhere herein (see the section in which the corresponding method is described) and a computer-implemented algorithm for carrying out a comparison as specified elsewhere herein (see the section in which the corresponding method is described).

In a preferred embodiment, the device also comprises a detection agent which specifically binds to a KIM-1 adapted for determining the amount of KIM-1. Preferably, the evaluation unit (b) comprised by said device is for calculating a ratio of L-FABP to KIM-1 and for comparing the determined ratio with a reference ratio whereby it can be assessed whether said subject is susceptible to the administration of nephrotoxic medication, said unit comprising a database with reference ratios values derived from a subject or group of subjects as defined elsewhere herein and a computer-implemented algorithm for carrying out a comparison as specified elsewhere herein (see the section in which the corresponding method is described).

The term "device" as used herein relates to a system comprising the aforementioned units operatively linked to each other as to allow the diagnosis or monitoring according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

Furthermore, a kit adapted for carrying out the method of the present invention is contemplated, said kit comprising a detection agent for the biomarker L-FABP as well as instructions for carrying out the said method.

In a preferred embodiment, the kit further comprises a detection agent for the biomarker KIM-1.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards for reference amounts as described elsewhere herein in detail.

All references cited in this specification are herewith incorporated by reference with respect to their entire disclosure content and the disclosure content specifically mentioned in this specification.

### EXAMPLES

### Example 1: Determination of the biomarkers KIM-1 and L-FABP

U-LFABP was determined by using the L-FABP ELISA kit from CMIC, Ltd, Japan. The test is based on an ELISA 2 step assay. L-FABP standard or urine samples are firstly treated with pre-treatment solution provided by the manufacturer, and transferred into a L-FABP antibody coated microplate containing assay buffer and incubated. During this incubation, L-FABP in the reaction solution binds to the immobilized antibody. After washing, the 2^{nd} antibody-peroxidase conjugate is added as the secondary antibody and incubated, thereby forming sandwich of the L-FABP antigen between the immobilized antibody and conjugate antibody. After incubation, the plate is washed and substrate for enzyme reaction is added, color develops according to the L-FABP antigen quantity. The L-FABP concentration is determined based on the optical density. The measurement range of the kit is 3 to 400 ng/ml.

Human KIM-1 was determined by the human KIM-1 (catalogue number DY 1750) ELISA development kit from R&D systems, containing a capture antibody (goat anti-human TIM-1) and a detection antibody (biotinylated goat anti-human TIM 1). A seven point standard curve using 2 fold serial dilutions in reagent diluent provided by the manufacturer, and a high standard of 2000 pg/ml is recommended.

### Example 2: Patient cohorts

Healthy subjects: A total of 149 clinically healthy individuals (51 males, mean age 40 years (range 20 to 52 years) and 97 females, mean age 41 years (range 18 to 56 years) were included into the study as a control group. The subjects had normal blood pressure, a normal electrocardiogram, no evidence of diabetes mellitus as measured by fasting glucose levels and glucose tolerance test. These individuals had no history of kidney disease or disease of the lower urinary tract and their kidney function was within normal as measured by creatinine levels within normal. In addition, they had no obvious cardiac disease and their NT-pro BNP levels were within the normal range of the test (below 125 pg/ml), in addition they did not exhibit risk factors for coronary artery disease such as smoking. All individuals were anti-HIV negative.

Patients with type 2 diabetes mellitus: A total of 52 patients with type 2 diabetes mellitus and microalbuminuria (30 to 300 mg/24 h), but unimpaired kidney function as assessed by creatinine levels within the normal range were also included into the study. There were 41 males and 11 females, mean age 58 years. All patients were not treated with ACE inhibitors, Angiotensin receptor inhibitors, aldosterone antagonists or other diuretics. None of the patients had established cardiovascular disease and their NT-pro BNP levels were within the normal range (below 125 pg/ml). All patients were anti-HIV negative.

Patients with heart failure: A total of 44 patients with established heart failure were also included into the study, 25 males, 19 females, mean age 62,1 years. All patients had systolic heart failure as indicated by a left ventricular ejection fraction below 40% as measured by echocardiography. All patients were on ACE inhibitors, none of the patients had diabetes mellitus as indicated by normal fasting blood glucose levels and a normal glucose tolerance test. Median NT-pro BNP levels were 600 pg/ml confirming impaired cardiac function as assessed by echocardiography. All patients were clinically stable and the kidney function was within normal as indicated by creatinine levels within the normal range. All patients were anti-HIV negative.

HIV infected patients: A total of 300 patients with established HIV infection were included into the study. They were 280 males and 20 females, mean age 48 years (range: 36 - 67 years). Except 20 patients, all patients were on antiviral therapy. Antiviral therapy consisted of a various combinations of antivirals of the following classes: Nucleoside reverse transcriptase inhibitors, Nucleotide reverse transcriptase inhibitors (Tenofovir), Non-nucleoside reverse transcriptase inhibitors (NNRTIs) and protease inhibitors (PIs):

### Protease inhibitors (trade names and active ingredients)

Crixivan^{®}= Indinavir
Kaletra^{®} = Lopinavir and Ritonavir
Reyataz^{®} = Atzanavir
Telzir^{®}= Fosanprenavir
Invirase^{®} = Saquinavir
Prezistza^{®} = Duranavir

### NNRTIs

Sustiva^{®} = Efavirenz
Emtriva^{®} = Emtricabine

### NRTIs

Viramune^{®} = Neviparin
Kivexa^{®} = Abacavir und Lamivudine
Combivir^{®} = Idovudine und Lamivudine
Retrovir^{®} = Zidovudine
Videx^{®} = DDI
Epivir^{®} = Lamivudine
Ziagenavir^{®} = Abacavir

### Nucleotide reverse transcriptase inhibitors

Truvada^{®} = Tenofovir (and Emtricabine)
Viread ^{®}= Tenofovir

Antiviral therapy was associated with HIV-RNA levels below the detection limit in all but 68 patients (below 0.05 copies HIV-RNA). In patients lacking antiviral therapy HIV-RNA levels were below the detection limit of the test in 1 patient. All patients had creatinine levels within the normal range indicating that their kidney function was not impaired. None of the patients had established cardiovascular disease and the NT-pro BNP level was within the normal range (NT-pro BNP below 125 pg/ml) with the exception of 24 patients (NT-pro BNP above 125 pg/ml). Of these 24 patients, 21 patients had NT-proBNP levels between 125 and 300 pg/ml. Only three patients had NT-proBNP levels similar to those patients with (stable) heart failure (539, 654 and 1014 pg/ml). Diabetes mellitus type 2 was established in 29 patients, 30 patients were coinfected with the hepatitis C virus as indicated by positive anti-HCV tests.

Serum and urine samples were obtained from all subjects/patients, and were kept at least - 20°Celsius until used.

### Example 2: L-FABP and KIM-1 levels

Results for the different subgroups are given in Table 1:

**Table 1: Results are given as median, in addition the 25th and 75th percentiles are given**

| Patient group | U-L-FABP | KIM- 1 |
|---|---|---|
| | µg/g creatinine | µg/g creatinine |
| | | |
| Healthy subjects | 3.78 | 0.331 |
| N = 149 | 2,72 - 5.44 | 0.191 - 0.491 |
| | | |
| Diabetes mellitus type 2 | 5.51 | 0.314 |
| N = 51 | 3.61 - 12.15 | 0.136 - 0.66 |
| | | |
| Heart failure | 7.664 | 0.574 |
| N = 44 | 5,416 - 11.447 | 0.314 - 0.840 |
| | | |
| HIV infected | 4.864 | 0.420 |
| N = 300 | 2.374 - 9.858 | 0.210 - 0.670 |

As it can be derived from Table 1, markers of tubular damage (U- L-FABP) and tubular repair (KIM-1) were in average higher in HIV infected persons than in normal subjects. When compared with type 2 diabetes mellitus they had slightly lower U-L-FABP levels and slightly higher KIM 1 concentrations. Heart failure patients had highest U-L-FABP and KIM-1 levels, however clinical heart failure was not present in HIV infected persons and the number of HIV infected persons with elevated NT-pro >BNP levels was low.

In order to elucidate the effect of antiviral drugs on kidney damage markers, the effect of different classes of drugs was assessed. They were in addition compared to albumin excretion in urine, a method currently used to assess kidney damage. Results are summarized in Table 2.

Table 2: Markers of kidney damage in HIV infected individuals stratified by treatment classes. Data are given as median and also the 25^{th} and 75^{th} percentile are given.

### Abbreviations:

PI: Protease Inhibitor
NNRTI: Non-nucleoside reverse transcriptase inhibitor
RTI: Reverse transcriptase inhibitor

| Patient group | Albumine | U-L FABP | KIM 1 |
|---|---|---|---|
| | mg/g Crea | ug/g Crea | ug/g crea |
| no treatment | 5.356 | 5.902 | 0.255 |
| N = 20 | 2.407 - 8.210 | 0.000 - 7.21 | 0.163 -0.499 |
| | | | |
| PI | 4.931 | 4.902 | 0.457 |
| N = 135 | 2.913 -10.796 | 2,8 - 10.335 | 0.297 - 0.713 |
| | | | |
| NNRTIs | 4.321 | 4.290 | 0.384 |
| N = 73 | 2.431 - 8.721 | 0.0 - 9.583 | 0.147 - 0.525 |
| | | | |
| Nucleoside RTIs | 4.909 | 4.0902 | 0.465 |
| N = 264 | 2.647 - 10.239 | 2.535 - | 10.824 0.262 - 0.710 |
| | | | |
| Nucleotide RTIs | 5.006 | 6.653 | 0.532 |
| N = 98 | 2.708 - 9.775 | 3.598-13.512 | 0.299 - 0.761 |

Please note: The majority of patients was treated with various antiviral medicaments belonging to various classes (combination therapies). Therefore, the sum of the patients in table 2 exceeds the number of patients (N= 300) analyzed in this study. As can also be derived from Table 2, the majority of patients with HIV infection received nucleoside reverse transcriptase inhibitors (N = 264).

As can be seen from the table, antiviral therapy reduced tubular damage (U-L-FABP) and increased tubular repair (KIM-1) except in patients with nucleotide reverse transcriptase inhibitors (tenofovir). Patients treated with NNRTIs and Nucleoside RTIs had the lowest median level of L-FABP.

As set forth above, patients treated with a nucleotide reverse transcriptase inhibitor (Tenofovir) had the highest level of L-FABP, a marker of tubular damage. Therefore, it was assessed whether the choice of combinational therapy affects tubular damage. In particular, it was assessed whether PIs and NNRTIs affect these markers in patients treated with tenofovir.

Table 3 shows the level of L-FABP, KIM-1 and the ratio of L-FABP to KIM-1 in patients treated with a Protease inhibitor and Tenofovir. Table 4 shows the level of L-FABP, KIM-1 and the ratio of L-FABP to KIM-1 in patients treated with a NNRTI and Tenofovir.

**Table 3: Tenofovir + Protease inhibitor**

| | ug L-FABP/ g Crea | ug KIM-1/g Crea | L-FABP/KIM-1 Ratio |
|---|---|---|---|
| n | 38 | 38 | 38 |
| perc(25) | 2.438 | 0.351 | 5.407 |
| perc(50) | 6.549 | 0.554 | 10.640 |
| perc(75) | 14.538 | 0.785 | 28.217 |

**Table 4: Tenofovir + NNRTI**

| | ug L-FABP/ g Crea | ug KIM-1/ g Crea | L-FABP/KIM-1 Ratio |
|---|---|---|---|
| n | 11 | 11 | 11 |
| perc(25) | 4.667 | 0.106 | 10.862 |
| perc(50) | 9.765 | 0.323 | 43.961 |
| perc(75) | 16.118 | 0.510 | 102.523 |

As it can be seen from the table, the level of L-FABP was higher in patients treated with Tenofovir and a NNRTI than in patients treated with Tenofovir and a PI. The increased levels of L-FABP indicate increased tubular damage and an increased risk of acute kidney injury. Interestingly, the levels of KIM-1 in patients treated with Tenofovir and a NNRTI are lower than in patients treated with Tenofovir and a PI indicating decreased renal repair in Tenofovir/NNRTI treated individuals.

Moreover, the ratio of L-FABP to KIM-1 is significantly larger in patients treated with Tenofovir and a NNRTI than in patients treated with Tenofovir and PI. E.g., the median ratio of L-FABP to KIM-1 is 10.64 in the Tenofovir/PI group, but 43.96 in the Tenofovir/NNRTI group. Increased tubular damage (as indicated by increased levels of L-FABP) in combination with decreased renal repair (as indicated by decreased levels of KIM-1) indicate an increased risk of, e.g., acute kidney injury and chronic renal failure.

### Example 4: Case Studies

The amounts of the various biomarkers in individual patients are shown in Tables 5 and 6

**Table 5: Patients treated with Tenofovir and a NNRTI**

| Patient | µg L-FABP/g Creatinine | µg KIM-1/g Creatinine | L-FABP/KIM-1 ratio | Serum Creatinine [mg/dl] | Treated with |
|---|---|---|---|---|---|
| 5959 | 14,196 | 0,323 | 43,96 | 0,94 | Epivir, Viread, Viramune |
| 6770 | 9,195 | 0,072 | 127,14 | 1,01 | Truvada, Sustiva |
| 7932 | 10,880 | 0,140 | 77,90 | 0,92 | Truvada, Sustiva |
| 11743 | 3,717 | 0,582 | 6,39 | 1,07 | Truvada, Viramune |
| 13059 | 4,478 | 0,349 | 12,82 | 0,97 | Truvada, Sustiva |
| 13063 | 0,000* | 0,474 | 0,00 | 0,95 | Combivir, Viramune |
| 13802 | 222,896 | 0,002 | 148586 | 1 | Epivir, Viread, Sustiva, Fuzeon |
| 13953 | 4,856 | 0,545 | 8,90 | 1,18 | Epivir, Viread, Sustiva |
| 13976 | 74,764 | 0,054 | 1379,64 | 0,9 | Truvada, Sustiva |
| 14458 | 18,040 | 0,739 | 24,40 | 1,13 | Truvada, Viramune |
| 14661 | 9,765 | 0,164 | 59,61 | 1,03 | Truvada, Sustiva |

| | | | | | |
|---|---|---|---|---|---|
| *Not detectable | | | | | |

As it can be seen from the table, patients treated with tenofovir and a NNRTI have statistically the highest concentration of U-L-FABP indicating tubular damage. Thus, this combination therapy is not first choice in case of kidney disease specifically in case of impaired repair as illustrated by low levels of KIM 1. A typical case is case 13802, while case 11743 requires no discontinuation of this drug combination.

**Table 6: Patients treated with Tenofovir and a PI**

| Patient | µg L-FABP/g Creatinine | µg KIM-1/g Creatinine | L-FABP/KIM-1 ratio | Serum Creatinine [mg/dl] | Treated with |
|---|---|---|---|---|---|
| 537 | 0,000 | 1,313 | 0,00 | 0,98 | Combivir, Viread, Invirase |
| 2308 | 0,000 | 0,055 | 0,00 | 1,04 | Kivexa, Viread, Kaletra, Invirase |
| 2322 | 3,182 | 0,573 | 5,55 | 1,09 | Viread, Trizivir, Kaletra |
| 2573 | 18,215 | 1,011 | 18,02 | 1,06 | Truvada, Kaletra |
| 4318 | 70,564 | 1,501 | 47,00 | 1,02 | Viread, Ziagen, Kaletra |
| 4897 | 0,000 | 0,545 | 0,00 | 0,96 | Retrovir, Viread, Kaletra, Invirase |
| 5102 | 1,473 | 0,737 | 2,00 | 1,07 | Truvada, Telzir |
| 5281 | 0,000 | 0,311 | 0,00 | 1,24 | Viread, Ziagen, Reyataz |
| 5406 | 4,452 | 0,520 | 8,57 | 0,92 | Truvada, Kaletra |
| 5549 | 22,144 | 0,662 | 33,43 | 0,96 | Kivexa, Viread, Kaletra |
| 5733 | 27,607 | 0,524 | 52,64 | 0,99 | Kivexa, Viread, Kaletra |
| 5773 | 3,455 | 0,102 | 34,01 | 0,76 | Combivir, Viread, Reyataz |
| 5957 | 9,044 | 0,808 | 11,19 | 0,98 | Viread, Trizivir, Kaletra, Invirase |
| 6092 | 22,849 | 2,398 | 9,53 | 1,26 | Viread, Trizivir, Kaletra |
| 6421 | 8,936 | 0,352 | 25,39 | 1,07 | Viread, Emtriva, Kaletra |
| 6921 | 5,477 | 0,544 | 10,07 | 1,41 | Truvada, Kaletra |
| 8643 | 1,841 | 0,350 | 5,26 | 0,92 | Viread, Trizivir, Reyataz |
| 9004 | 2,497 | 0,098 | 25,59 | 1,04 | Truvada, Kaletra |
| 9353 | 5,995 | 0,720 | 8,33 | 1,28 | Kivexa, Viread, Reyataz |
| 9505 | 0,000 | 0,618 | 0,00 | 1,21 | Truvada, Kaletra |
| 10547 | 4,902 | 1,196 | 4,10 | 1 | Truvada, Kaletra |
| 10547 | 16,494 | 2,345 | 7,03 | 0,97 | Truvada, Kaletra |
| 12561 | 11,162 | 0,331 | 33,75 | 1,19 | Epivir, Viread, Kaletra |
| 13210 | 14,688 | 1,184 | 12,41 | 1,09 | Truvada, Telzir |
| 13562 | 0,000 | 0,404 | 0,00 | 0,92 | Viread, Emtriva, Kaletra |
| 13639 | 6,873 | 0,563 | 12,21 | 0,85 | Truvada,Reyataz |
| 14374 | 11,688 | 1,607 | 7,27 | nd | Truvada,Kaletra |
| 14709 | 6,944 | 0,688 | 10,09 | 0,8 | Truvada,Invirase |
| 14766 | 6,936 | 0,429 | 16,19 | 1,04 | Truvada,Kaletra |
| 14835 | 3,718 | 0,121 | 30,84 | 0,92 | Truvada,Invirase |
| 14850 | 23,536 | 0,761 | 30,92 | 0,94 | Truvada,Kaletra |
| 15329 | 37,085 | 0,570 | 65,09 | 0,81 | Truvada,Kaletra |
| 15623 | 14,388 | 1,733 | 8,30 | 0,96 | Truvada,Reyataz |
| 16590 | 7,095 | 0,299 | 23,77 | 0,95 | Truvada,Kaletra |
| 16984 | 0,000 | 0,383 | 0,00 | 0,71 | Truvada,Kaletra |
| 18564 | 29,630 | 0,170 | 174,63 | 0,87 | Truvada,Reyataz |
| 600769 | 6,226 | 0,353 | 17,62 | 1,07 | Truvada,Invirase |

With regard to kidney damage, and considering the results of U-LFABP and KIM-1, the combination therapy of tenofovir with a PI is advantageous over a combination of tenofovir with a NNRTI. While the combination of tenofovir with a PI is generally preferably over the teneofovir/NNRTI combination (lower U-L-FABP and higher KIM-1)(less tubular damage and improved repair) individual cases may still require consideration (and testing).

### Example 5: Effects of HIV comorbidities on urine biomarkers

In a separate analysis the effect of comorbitities (Diabetes mellitus, HCV coinfection) was analysed. The results are shown in Table 7.

**Table 7**

| Patient group | Albumine | U-LFABP | KIM-1 |
|---|---|---|---|
| | Mg/g Crea | ug/g Crea | ug/g Crea |
| | | | |
| HIV total | 5.081 | 4,864 | 0,420 |
| N = 300 | 2,662 - 10.239 | 2,374 - 9,858 | 0,21 - 0,67 |
| | | | |
| HCV coinfection | 7,374 | 8,552 | 0,785 |
| N = 30 | 3,795 -11,715 | 3,384 - 22,379 | 0,383 -1,125 |
| | | | |
| Diabetes mellitus as | | | |
| Comorbitity | 11,450 | 8.90 | 0,538 |
| N = 29 | 3,759 - 28,092 | 4,06 - 19,73 | 0,331 - 0,750 |

As can be seen from Table 7, HCV and diabetes mellitus as comorbidities significantly increase kidney damage markers in urine, indicating that these comorbidities warrant intense monitoring.

### Example 6

A 39 year old male patient who is HIV positive for 10 years has been started with Atripla^{®} for reasons of convenience one month ago. Atripla is fixed dose combination of emtricitabine (a nucleoside reverse transcriptase inhibitor) tenofovir (a nucleotide reverse transcriptase inhibitor), and efavirenz (a non-nucleoside reverse transcriptase inhibitor). Before switching to Atripla^{®} U-L-FABP was 4,2 µg/g creatinine and KIM-1 was 0,42 µg/g creatine, his values have now increased to U L FABP 21,2 µg/g creatinine and KIM 1 decreased to 0,32 µg/creatinine, in terms of virological response HIV-RNA remained below 0,05 copies/microliter. He reports no symptoms. Kidney function tests did not worsen and his serum creatinine moved from 0,7 mg/dl to 0,8 mg/ml. The patient is informed that the new drug schedule results in increased tubular damage. Therapy is continued with Truvada^{®} (combination of tenofovir and emtricabine) and ritonavir (a protease inhibitor).

### Example 7

A 29 year old male drug addict continuously on use of illegal drugs, known to be HIV positive for 6 years, presents with loss of vision of his right exe, he reports not having taken antiviral drugs for the last 6 months, his CD 4 count is measured with 56 CD 4 cells/Microliter, HIV-.RNA was 3,6 x 1000 copies per microliter. He is diagnosed with CMV infection. U-L-FABP is 5,3 µg/g creatinine and KIM-1 is 0,4 µg/g creatinine. He is treated with gancyclovir and an antiviral drug combination not containing tenofovir and a NNRTI after having performed drug resistance testing. He was hospitalised for treatment and was closely monitored for HIV-RNA to determine virological response, CD 4 counts, CMV-DNA for treatment response to CMV infection as well as creatinine, U L FABP and KIM 1. He is promised to be switched to Atripla a convient one time per day drug after HIV-RNA has become undetectable, CD 4 counts have increased and U LFABP and KIM 1 would permit this combination therapy.

### Example 8

A 39 old HIV infected patient positive for HIV for the past 5 years and on Atripla for the past month reports reduced urine volume, on presentation creatine is 3,2 mg/ml (0,8 mg/dl 6 months before), HIV-RNA is undetectable, CD4 count is 280 cells/microliter. U-LFABP is 205 µg/g creatinine and Kim-1 is 0,1 µg/g creatinine. He is diagnosed with acute kidney injury and antiviral therapy is discontinued, in the next few days creatinine improved and the patient did not require dialysis, also U LFABP decreases to 25 ug/g creatinine and KIM 1increased to 1,6 g/g creatinine indicating reduction of tubular damage associated with increased repair. Therapy is continued without the administration of efavirenz.

### General conclusion:

Treatment of HIV disease using antiviral drugs is complex and requires complex considerations. The primary goal of HIV infection is suppression of viral replication with HIV-RNA below the level of detection of current assays (below 0,05 copies per microliter). This goal can be achieved using different drugs combinations, viral resistance in this context may limit the choice of drugs. As outlined in the introduction several additional components need to be considered in the treatment of HIV specifically when recognizing that patients experience increased survival and thus reach an age were common diseases become more frequent and lead to premature death if certain aspects are not considered. In this case, the early identification of underlying causes of acute and chronic renal disease using U-L-FABP und KIM-1 (as marker for tubular damage) allow to further adopt treatment strategies and to identify preferred treatment schedules as well as monitoring of certain drug combinations allowing further treatment adaptation. In particular, the use of the biomarkers U-L-FABP and KIM-1 allows for optimizing antiretroviral therapy with respect to adverse side effects on kidney function. Thereby, it is possible to reduce the risk of acute kidney injury and chronic kidney disease.

## Claims

1. A method for assessing whether an HIV infected subject who is or who will be administered a nucleotide reverse transcriptase inhibitor (NTRTI) is susceptible to the administration of a non-nucleoside reverse transcriptase inhibitor (NNRTI), said method comprising
a) determining the amount of L-FABP (liver fatty acid binding protein) in a sample from said subject, and
b) comparing the amount of L-FABP to a reference amount, whereby it is assessed whether said subject is susceptible to the administration of a non-nucleoside reverse transcriptase inhibitor (NNRTI).

2. The method of claim 1, wherein said reference amount is derived from an HIV infected subject of from a group of HIV infected subjects known to be susceptible to the administration of a non-nucleoside reverse transcriptase inhibitor (NNRTI), and wherein an essentially identical or a decreased amount of L-FABP relative to the reference amount is indicative for a subject being susceptible the administration of a non-nucleoside reverse transcriptase inhibitor (NNRTI).

3. The method of claim 1, wherein said reference amount is derived from an HIV infected subject or from a group of HIV infected subjects known not to be susceptible to the administration of a non-nucleoside reverse transcriptase inhibitor (NNRTI), and wherein an essentially identical or an increased amount of L-FABP relative to the reference amount is indicative for a subject not being susceptible to the administration of said non-nucleoside reverse transcriptase inhibitor (NNRTI).

4. The method of claim 3, wherein a subject not being susceptible to the administration of said non-nucleoside reverse transcriptase inhibitor (NNRTIs) is susceptible to the administration of a protease inhibitor.

5. The method of any one of claims 1 to 4, wherein said nucleotide reverse transcriptase inhibitor is tenofovir.

6. The method of any one of claims 1 to 5, wherein said non-nucleoside reverse transcriptase inhibitor is selected from the group consisting of efavirenz, nevirapine, delavirdine and etravirine.

7. The method of any one of claims 1 to 6, wherein said sample is blood, plasma or serum.

8. The method of any one of claims 1 to 6, wherein said sample is urine.

9. The method of claim 8, wherein the method further comprises the determination of the amount of KIM-1 (kidney injury molecule 1) in a sample from said subject, the calculation of a ratio of the amount of L-FABP to the amount of KIM-1, and the comparison of the, thus, determined ratio to a reference ratio.

10. A method for monitoring a therapy comprising the administration of nucleotide reverse transcriptase inhibitor in an HIV infected subject, said method comprising the steps of
a) determining the amount of L-FABP (liver fatty acid binding protein) in a first sample from said subject,
b) determining the amount of L-FABP (liver fatty acid binding protein) in a second sample from said subject, said second sample being obtained after said first sample, and
c) comparing the amount of L-FABP in said first sample to the amount in said second sample, whereby said therapy comprising the administration of nucleotide reverse transcriptase inhibitor in said subject is monitored.

11. The method of claim 8, wherein said therapy further comprises the administration of a non-nucleoside reverse transcriptase inhibitor (NNRTI).

12. A method for assessing whether an HIV infected subject who is or will be administered a nucleotide reverse transcriptase inhibitor (NTRTI) is susceptible to nephrotoxic medication, said method comprising the steps of
a) determining the amount of L-FABP (liver fatty acid binding protein) in sample from said subject,
b) comparing the amount of L-FABP to a reference amount, and
c) assessing whether said subject is susceptible to nephrotoxic medication.

13. Use of the biomarker L-FABP or a detection agent that specifically binds thereto in a sample from an HIV infected subject who is or who will be administered a nucleotide reverse transcriptase inhibitor (NTRTI) for assessing whether said subject is susceptible to the administration of a non-nucleoside reverse transcriptase inhibitor (NNRTI), or for monitoring a therapy comprising the administration of a nucleotide reverse transcriptase inhibitor, or for assessing whether said subject is susceptible to the administration of nephrotoxic medication.

14. A device adapted for carrying out the method of any one of claims 1 to 9, comprising
a) an analyzing unit comprising a detection agent which specifically binds to L-FABP, said unit being adapted for determining the amount of L-FABP in a sample of a subject who is or who will be administered a nucleotide reverse transcriptase inhibitor (NTRTI); and
b) an evaluation unit for comparing the determined amount with a reference amount, whereby it can be assessed whether said subject is susceptible to the administration of a non-nucleoside reverse transcriptase inhibitor (NNRTI), said unit comprising a database with reference amount values derived from a subject or group of subjects as defined in claim 2 and/or 3 and a computer-implemented algorithm for carrying out a comparison as specified in claim 2 and/or 3.

15. A kit adapted for carrying out the method of any one of claims 1 to 12 comprising a detection agent for the biomarker L-FABP, as well as instructions for carrying out the said method.
